# EUROPÄISCHE PATENTSCHRIFT

(11) **EP 0 948 939 B1**
(45) Veröffentlichungstag und Bekanntmachung des Hinweises auf die Patenterteilung: **12.05.2004**
(21) Anmeldenummer: 99106097.1
(22) Anmeldetag: 26.03.1999
(51) Int. Cl.: A61B 17/88, A61B 17/70

(54) **Pedikelschraube und Montagehilfe dafür**
Pedicle screw and mounting tool
Vis pédiculaire et dispositif de montage

(30) Priorität: 09.04.1998 DE 29806563 U
(43) Veröffentlichungstag der Anmeldung: 13.10.1999
(73) Patentinhaber: Stryker Trauma GmbH, 24232 Schönkirchen (DE)
(72) Erfinder: Lutz, Christian, 24796 Bovenau (DE)
(74) Vertreter: Liebetanz, Michael, Dipl.-Phys.

(56) Entgegenhaltungen:
- WO-A-95/14437
- DE-A- 4 238 339
- US-A- 5 649 931

## Beschreibung

Die Erfindung bezieht sich auf eine Pedikelschraube und eine Montagehilfe dafür nach dem Oberbegriff des Anspruchs 1. Der nächstliegende Stand der Technik ist DE-A-4 238 339.

Zur Reponierung von Wirbeln der menschlichen Wirbelsäule, zur Distraktion der Wirbelsäule, zur Stabilisierung von Wirbeln und dergleichen ist bekannt, sogenannte Pedikelschrauben zu verwenden. Sie werden in die Pedikel der Wirbel eingeschraubt und weisen einen Kopf auf, der mit geeigneten Vorkehrungen verbunden wird, beispielsweise einem Stabilisierungssystem, mit Distraktionsstäben und dergleichen. Es ist bekannt, Pedikelschrauben mit einem ringartigen Kopf zu versehen, der an den Stirnseiten eine Zahnung aufweist. Es ist jedoch auch bekannt, den Schraubenkopf gabelartig zu gestalten, damit er einen Distraktionsstab, einen Verbindungsstab oder dergleichen aufzunehmen in der Lage ist. Zur Festlegung des Stabes im gabelartigen Kopf kann ein geeignetes Schraubelement mit dem Kopf in Eingriff gebracht werden. Das Schraubelement kann hutartig sein und auf Außengewindeabschnitte des Kopfes aufgeschraubt werden. Es ist jedoch auch bekannt, den Kopfabschnitten ein Innengewinde zuzuordnen zur Aufnahme einer Madenschraube oder dergleichen, mit welche der eingelegte Stab im Kopf festgelegt wird.

Es ist ferner bekannt, zur einfacheren Handhabung der Pedikelschraube diese mit einem Kopf zu versehen, der relativ zum Schaft beweglich ist, vorzugsweise in allen Richtungen schwenkbar. Zu diesem Zweck kann das Kopfende des Schaftes kugelig ausgeführt werden, wobei die Kugelfläche mit einer sphärischen Lagerfläche im Kopf zusammenwirkt. In diesem Fall ist erforderlich, nach erfolgter Einstellung von Schaft und Kopf zueinander diese Winkelstellung beizubehalten. Dies kann beispielsweise dadurch geschehen, daß der vom Kopf aufgenommene Stab gegen den kugeligen Kopf des Schaftes gepreßt wird, wodurch die Teile in ihrer Lage zueinander festgelegt sind.

Bei der Versorgung werden im allgemeinen zuerst die Pedikelschrauben eingedreht. Anschließend erfolgt das Einlegen des Stabes. Hierbei müssen jedoch unter Umständen erhebliche Kräfte aufgewendet werden, um den Stab in den gabelartigen Kopf hineinzubewegen. Die Einführkraft muß so lange aufrechterhalten werden, bis mit Hilfe des Schraubelements ein Herausgleiten aus der Aufnahme im Kopf nicht mehr möglich ist. Hierzu bedarf es erheblicher Geschicklichkeit und auch eines erheblichen Kraftaufwandes seitens des Chirurgen.

Der Erfindung liegt daher die Aufgabe zugrunde, die Pedikelschraube so auszubilden und eine Montagehilfe derart auszuführen, daß das Einlegen und Festlegen von Stäben in den Pedikelschraubenköpfen erleichtert ist.

Diese Aufgabe wird durch die Merkmale des Anspruchs 1 gelöst.

Ein wesentliches Merkmal der Erfindung besteht darin, daß die Kopfabschnitte auf der Außenseite eine Ausnehmung aufweisen. Die Ausnehmung dient zur Aufnahme von Greifabschnitten, die am Ende von Armen ausgebildet sind, die ihrerseits mit einem Schaft verbunden sind. Der Schaft ist gleitend, jedoch drehfest in einer Hülse geführt, und zwischen Hülse und Schaft wirkt ein Verstellmechanismus, der bei Betätigung den Schaft relativ zur Hülse verschiebt.

Die Greifabschnitte erfassen von der Außenseite den Pedikelschraubenkopf, so daß der Kopf mit Hilfe des beschriebenen Verstellmechanismus in Richtung Hülse bewegt werden kann. Der Stab ist zuvor zwischen die Arme eingeführt worden, bevor der Pedikelschraubenkopf erfaßt wurde. Wird nunmehr der Schraubenkopf in Richtung Hülse bewegt, drückt die Hülse den Stab automatisch zwischen die Kopfabschnitte in die Ausnehmung hinein.

Der Schaft weist einen axialen Durchgang auf, durch den ein relativ dünner Schaft eines Drehwerkzeugs hindurchgeführt werden kann. Mit Hilfe des Drehwerkzeugs, an dem bereits eine entsprechende Feststellschraube angebracht ist, kann nunmehr die Madenschraube mit den Innengewindeabschnitten des Kopfes verschraubt werden. Dadurch drückt die Schraube den Stab gegen den Boden der Aufnahme. Bei ausreichend hoher Anzugskraft kann daher auch bei einer zweiteiligen Ausführung einer Pedikelschraube, d.h. mit einem schwenkbar zum Schraubenschaft gelagerten Kopf, eine Fixierung in der eingestellten Winkellage erreicht werden.

Das Zusammenwirken der Arme mit der Außenseite des Pedikelschraubenkopfes kann in irgendeiner geeigneten Art und Weise erfolgen. Eine Ausgestaltung der Erfindung sieht hierzu vor, daß die Außenkontur der Kopfabschnitte konvex gerundet ist und die Ausnehmung eine achsparallel plan verlaufende Bodenfläche aufweist, die in Umfangsrichtung des Kopfes in die Außenkontur übergeht. Sind die Greifmittel zum Beispiel klauenartige Ansätze an den Armen, können diese seitlich zum Kopf in Stellung gebracht und anschließend durch eine Bewegung seitwärts zum Kopf ausgerichtet werden. Wird nunmehr relativ zum Kopf eine Zugkraft auf die Arme aufgebracht, erfassen die klauenartigen Abschnitte die obere Wand der Ausnehmung und ziehen dadurch die Pedikelschraube in Richtung der Hülse der Montagehilfe.

Der Verstellmechanismus kann ebenfalls in bekannter Art und Weise ausgeführt sein. Besonders vorteilhaft ist eine Ausführung derart, daß der Schaft am hinteren Ende ein Außengewinde aufweist, auf das eine eine Innengewindebohrung aufweisende Handhabe in Anlage an das hintere Ende der Hülse aufschraubbar ist.

Um eine Führung für das Einlegen des Stabes oder Drahtes in die Aufnahme des Pedikelschraubenkopfes zu erhalten, ist nach einer Ausgestaltung der Erfindung vorgesehen, daß das vordere Ende der Hülse eine diametrale Ausnehmung aufweist, die an die Umfangskontur des Stabes angepaßt ist.

Ein Ausführungsbeispiel der Erfindung wird nachfolgend anhand von Zeichnungen näher erläutert.
- Fig. 1: zeigt eine Seitenansicht einer Montagehilfe nach der Erfindung.
- Fig. 2: zeigt einen Schnitt durch die Montagehilfe nach Fig. 1.
- Fig. 3: zeigt die Endansicht der Hülse der Montagehilfe nach Fig. 2.
- Fig. 4: zeigt in Seitenansicht eine Pedikelschraube für eine Montagehilfe nach den Figuren 1 bis 3.
- Fig. 5: zeigt einen Schnitt durch den Kopf der Pedikelschraube nach Fig. 4 entlang der Linie 5-5.

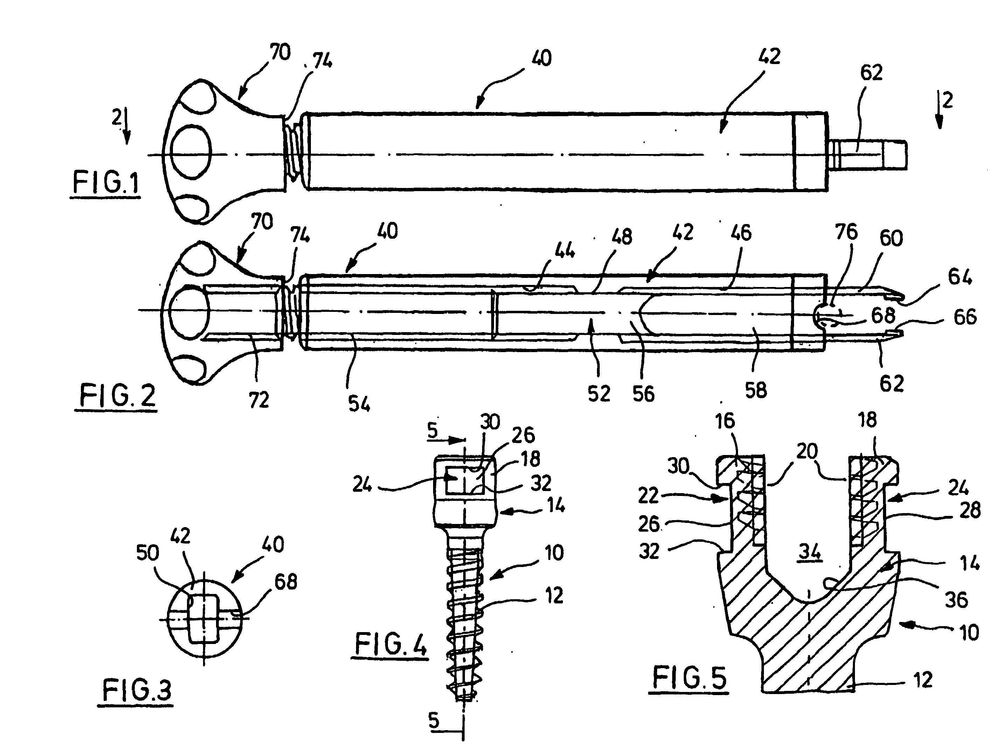

In den Figuren 4 und 5 ist eine Pedikelschraube 10 angedeutet. Sie weist einen Gewindeschaft 12 auf sowie einen Kopf 14. Der Kopf 14 ist gabelförmig, bestehend aus den Abschnitten 16, 18, die außen eine annähernd konvexe Kontur aufweisen. An der Innenseite sind Gewindeabschnitte 20 vorgesehen zur Aufnahme einer nicht gezeigten Feststellschraube in Form einer Madenschraube oder dergleichen. Wie erkennbar, ist eine Art Sägezahngewinde bei den Gewindeabschnitten 20 vorgesehen. Hierauf wird im einzelnen jedoch nicht eingegangen.

An der Außenseite der Kopfabschnitte 16, 18 ist eine Ausnehmung 22 bzw. 24 geformt. Wie aus der Zusammenschau der Figuren 4 und 5 hervorgeht, weisen die Ausnehmungen 22, 24 einen ebenen Boden 26 bzw. 28 auf, der in Umfangsrichtung in die Außenkontur der Kopfabschnitte 16, 18 übergeht. Dadurch ist am unteren und oberen Ende der Ausnehmungen 22, 24 jeweils eine Schulter 30 bzw. 32 geformt.

Die Kopfabschnitte 16, 18 bilden eine Aufnahme 34 für einen nicht gezeigten Stab, wobei der Boden 36 der Aufnahme 34 gerundet ist. Der in die Aufnahme 34 eingelegte Stab kann mit Hilfe der nicht gezeigten Feststellschraube in der Aufnahme gehalten bzw. festgelegt werden.

In den Figuren 1 bis 3 ist eine Montagehilfe 40 dargestellt. Sie enthält eine längliche Hülse 42, die innen zylindrische Abschnitte 44 bzw. 46 aufweist, welche durch einen engeren Abschnitt 48 getrennt sind. Am rechten bzw. vorderen Ende der Hülse 46 ist diese mit einem ovalen Durchgang 50 versehen. Die Hülse 46 nimmt einen Schaft 52 auf, der im linken Bereich einen Außengewindeabschnitt 54 aufweist, in der Mitte einen im Durchmesser etwas kleineren zylindrischen Abschnitt 56 und rechts davon einen im Querschnitt ovalen Abschnitt 58, der annähernd komplementär zum Durchgang 50 nach Fig. 3 geformt ist. Mithin ist der Gewindeabschnitt 54 annähernd durch den zylindrischen Abschnitt der Hülse 44 geführt und der zylindrische Abschnitt 56 durch den Innenzylinderabschnitt 48 der Hülse. Der ovale Abschnitt 58 des Schaftes 52 sorgt für die Drehsicherung des Schaftes 52 in der Hülse 46.

Am rechten Ende in Fig. 2 sind am Schaftabschnitt 58 zwei parallele Arme 60, 62 angebracht, die am freien Ende Klauenabschnitte 64 bzw. 66 aufweisen. Die Hülse hat eine diametrale halbkreisförmige Ausnehmung 68, deren Achse senkrecht zur Ebene der Arme 60, 62 verläuft.

Der Außengewindeabschnitt 54 erstreckt sich nach links über die Hülse 42 hinaus. Auf diesen ist ein Knauf 70 aufgeschraubt. Zu diesem Zweck hat dieser einen Innengewindeabschnitt 72. Wird der Knauf 70 auf den Außengewindeabschnitt 54 aufgeschraubt, schlägt er mit seiner einen Endfläche 74 gegen die zugeordnete Endfläche der Hülse 42 und zieht bei weiterem Drehen den Schaft 52 nach links, wodurch sich die Arme 60, 62 in die Hülse 46 hineinbewegen. Sind die klauenartigen Greifabschnitte 64, 66 in Eingriff mit den Ausnehmungen 22, 24, ziehen sie die aufgenommene Pedikelschraube in Richtung Hülse 42. Ist zwischen die Arme 60, 62 ein Stab eingelegt, wie gestrichelt bei 76 in Fig. 2 angedeutet, wird der Stab 76 allmählich in die Aufnahme 34 des Kopfes 14 hineinbewegt.

Der Schaft 52 ist in seiner gesamten Länge durchbohrt, weist mithin einen axialen Durchgang auf, durch den ein relativ dünner Schaft eines Drehwerkzeugs hindurchgeführt werden kann. Der Schaft kann eine ebenfalls nicht gezeigte Feststellschraube aufnehmen, beispielsweise durch eine Steckverbindung. Die Kombination aus Schaftdrehwerkzeug und aufgenommener Feststellschraube wird durch den Schaft 52 hindurchgeführt. Die Feststellschraube kann dann mit Hilfe des Drehwerkzeugs in das Gewinde 20 des Kopfes 14 eingeschraubt werden und dadurch den Stab weiter in die Aufnahme 34 hineinbewegen bis zur Anlage an den Grund der Aufnahme 34. Anschließend kann die Montagehilfe 40 entfernt werden.

Die Anbringung einer Pedikelschraube an den Armen 60, 62 erfolgt derart, daß die Ansätze 64, 66 seitlich zum Kopf 14 in Höhe der Ausnehmungen 22, 24 bewegt werden. In gleicher Weise erfolgt das Trennen der Montagehilfe. Die Arme 60, 62 können etwas federn und leicht aufeinander zu vorgespannt sein, um die Pedikelschraube 10 nach der Aufnahme des Kopfes zu halten. Es ist auch möglich, die Montagehilfe 40 axial zur Pedikelschraube 10 auszurichten und die Arme 60, 62 auf den Kopf 14 zu schieben. Die Arme 60, 62 werden seitlich auseinander bewegt, bis die Ansätze 64, 66 in die Ausnehmung 22, 24 einschnappen.

## Patentansprüche

1. Pedikelschraube und Montagehilfe dafür, wobei die Pedikelschraube einen Gewindeschaft und einen von zwei beabstandeten Abschnitten gebildeten gabelartigen Kopf aufweist und wobei die Abschnitte zwischen sich eine Aufnahme für einen länglichen Stab bilden und an den einander zugekehrten Seiten Gewindeabschnitte vorgesehen sind, in die ein Gewindeelement einschraubbar ist zur Festlegung des Stabes am Kopf, wobei die Kopfabschnitte (16, 18) der Pedikelschraube (10) außen eine Ausnehmung (20, 22) aufweisen, die Montagehilfe (40) eine längliche Hülse (42) aufweist, in der ein Schaft (52) axial, jedoch drehgesichert verschiebbar ist, und der Schaft (52) einen axialen Durchgang aufweist für die Durchführung eines relativ dünnen Werkzeugschaftes vom hinteren Ende der Hülse (42) bis über das vordere Ende des Schafts (52) hinaus, **dadurch gekennzeichnet, daß** am vorderen Ende des Schafts (52) zwei parallel im Abstand angeordnete Arme (60, 62) angebracht sind, die mit einem vorderen Greifabschnitt (64, 66) in die Ausnehmungen (22, 24) des Kopfes (14) zur Übertragung einer Zugkraft eingreifen, und zwischen Hülse (42) und Schaft (52) ein Verstellmechanismus (54, 70) vorgesehen ist zur axialen Relativverstellung der beiden Teile

2. Pedikelschraube und Montagehilfe nach Anspruch 1, **dadurch gekennzeichnet, daß** die Außenkontur der Kopfabschnitte (16, 18) konvex gerundet ist und die Ausnehmung (22, 24) eine achsparallele plan verlaufende Bodenfläche (26, 28) aufweist, die in Umfangsrichtung des Kopfes (14) in die Außenkontur übergeht.

3. Pedikelschraube und Montagehilfe nach Anspruch 1 oder 2, **dadurch gekennzeichnet, daß** die Greifabschnitte von klauenartigen Ansätzen (64, 66) der Arme (60, 62) gebildet sind.

4. Pedikelschraube und Montagehilfe nach einem der Ansprüche 1 bis 3, **dadurch gekennzeichnet, daß** der Schaft (52) am hinteren Ende einen Außengewindeabschnitt (54) aufweist, auf den eine einen Innengewindeabschnitt (72) aufweisende Handhabe (70) in Anlage an das hintere Hülsenende aufschraubbar ist.

5. Pedikelschraube und Montagehilfe nach einem der Ansprüche 1 bis 4, **dadurch gekennzeichnet, daß** das vordere Ende der Hülse (42) eine diametrale Ausnehmung (68) aufweist, die an die Umfangskontur eines Stabes (76) angepaßt ist.

6. Pedikelschraube und Montagehilfe nach Anspruch 4 oder 5, **dadurch gekennzeichnet, daß** die Handhabe (70) knaufartig ausgebildet ist.

## Claims

1. A pedicle screw and an assembly aid therefore, wherein the pedicle screw comprises a threaded shank and a fork-like head formed by two distanced sections and wherein the sections between themselves form a receiver for an elongate rod and on the sides facing one another there are provided threaded sections into which a threaded element may be screwed for fastening the rod on the head, wherein the head sections (16, 18) of the pedicle screw (10) on the outside comprise a recess (20, 22) and the assembly aid (40) comprises an elongate sleeve (42) in which a shank (52) is axially but rotationally securely displaceable, and wherein the shank (52) comprises an axial passage for the leading through of a relatively thin tool shank from a rear end of the sleeve (42) until beyond the front end of the shank (52) **characterized in that** on a front end of the shank (52) there are attached two parallel arms (60, 62) arranged at a distance, which with a front gripping section (64, 66) engage into the recesses (22, 24) of the head (14) for transmitting a tension force and between the sleeve (42) and the shank (52) there is provided an adjusting mechanism (54, 70) for the axial relative adjustment of the two parts.

2. A pedicle screw and an assembly aid according to claim 1, wherein the outer contour of the head sections (16, 18) is convexly rounded and the recess (22, 24) comprises a planarly running floor surface (26, 28) parallel to the axis, which in the circumferential direction of the head (14) blends into the outer contour.

3. A pedicle screw and an assembly aid according to claim 1 or 2, wherein the gripping sections are formed by claw-like attachments (64, 66) of the arms (60, 62).

4. A pedicle screw and an assembly aid according to one of claims 1 to 3, wherein the shank (52) at the rear end comprises an outer threaded section (54) onto which a handle (70) comprising an inner threaded section (72) can be screwed on, bearing onto the rear end of the sleeve.

5. A pedicle screw and an assembly aid according to one of claims 1 to 4, wherein a front end of the sleeve (42) comprises a diametric recess (68) which is adapted to a circumferential contour of a rod (76).

6. A pedicle screw and an assembly aid according to claim 4 or 5, wherein the handle (70) is formed knob-like.

## Revendications

1. Vis pédiculaire et dispositif de montage, la vis pédiculaire présentant un fût fileté et une tête en de type fourchette formée de deux sections écartées et les sections formant entre elles un logement pour une tige longiligne et des sections filetées étant prévues sur les côtés tournés l'un vers l'autre, dans lesquelles un élément fileté peut être vissé pour la fixation de la tige à la tête, les sections de tête (16, 18) de la vis pédiculaire (10) présentant à l'extérieur un creux (20, 22), le dispositif de montage (40) présentant une douille longiligne (42) dans laquelle un fût (52) peut coulisser dans le sens axial mais sans pivotement possible et le fût (52) présentant une traversée axiale pour la traversée d'un fût d'outil relativement mince de l'extrémité arrière de la douille (42) jusqu'au-delà de l'extrémité avant du fût (52), **caractérisés en ce que** deux bras (60, 62) sont montés à l'extrémité avant du fût (52), parallèles et écartés, qui s'engagent par une section avant d'engagement (64, 66) dans les creux (22, 24) de la tête (14) pour la transmission d'une force de traction, et qu'entre la douille (42) et le fût (52) est prévu un mécanisme de réglage (54, 70) pour le réglage relatif axial des deux pièces.

2. Vis pédiculaire et dispositif de montage selon la revendication 1, **caractérisés en ce que** le contour extérieur des sections de tête (16, 18) est arrondi de façon convexe et le creux (22, 24) présente une surface de fond (26, 28) s'étendant parallèlement à l'axe de façon plane qui passe dans le contour extérieur dans le sens périphérique de la tête (14).

3. Vis pédiculaire et dispositif de montage selon la revendication 1 ou 2, **caractérisés en ce que** les sections d'engagement sont formées par des pièces rapportées de type griffe (64, 66) des bras (60, 62).

4. Vis pédiculaire et dispositif de montage selon l'une quelconque des revendications 1 à 3, **caractérisés en ce que** le fût (52) présente une section de filetage extérieur (54) sur l'extrémité arrière sur laquelle une poignée (70) présentant une section de filetage intérieur (72) peut être vissée en appui sur l'extrémité arrière de la douille.

5. Vis pédiculaire et dispositif de montage selon l'une quelconque des revendications 1 à 4, **caractérisés en ce que** l'extrémité avant de la douille (42) présente un creux diamétral (68) adapté au contour extérieur d'une tige (76).

6. Vis pédiculaire et dispositif de montage selon la revendication 4 ou 5, **caractérisés en ce que** la poignée (70) est de type « pommeau ».
